Europäisches Patentamt

**European Patent Office**

**Office européen des brevets**

(11) Publication number: **0 264 552**
**A2**

## (12) EUROPEAN PATENT APPLICATION

(21) Application number: **87111214.0**

(22) Date of filing: **04.08.87**

(51) Int. Cl.4: **B65B 9/10** , B65D 81/32 ,
B65D 75/48 , A61K 47/00

The title of the invention has been amended (Guidelines for Examination in the EPO, A-III, 7.3).

(30) Priority: **22.08.86 GB 8620425**

(43) Date of publication of application:
**27.04.88 Bulletin 88/17**

(84) Designated Contracting States:
**BE DE ES FR IT LU NL SE**

(71) Applicant: **AMERICAN CYANAMID COMPANY**
**1937 West Main Street P.O. Box 60**
**Stamford Connecticut 06904-0060(US)**

(72) Inventor: **Ramsey, Michael Peter**
**83 Hatherley Crescent**
**Portchester Hampshire(GB)**

(74) Representative: **Wächtershäuser, Günter, Dr.**
**et al**
**Tal 29**
**D-8000 München 2(DE)**

(54) Multi-compartment pack containing a pharmaceutical gel preparation.

(57) There is disclosed a two-compartment burstable seam sachet containing a sterile unit dosage of a Varidase gel for topical administration.

EP 0 264 552 A2

## STABLE PHARMACEUTICAL GEL PREPARATION

This invention relates to pharmaceutical preparations which are to be applied topically in the form of gels.

There are a number of therapeutic agents which are applied topically in the form of gels, and there are others which it is known could be usefully administered as gels but which have hitherto proved difficult to satisfactorily present in this form. Typically, a pharmaceutical gel for topical administration is prepared by forming a solution, usually in water, of the active therapeutic agent and a gel-forming polymer, and then adding an agent for gelation, which causes the solution to convert into a gel. Examples of useful gel-forming polymers are carboxy vinyl polymers of high molecular weight (above 1,000,000), such as those sold by B F Goodrich under the trade mark "Carbopol", aqueous solutions of which form gels when the pH of the solution is raised above about pH 3.5 by the addition of an inorganic base such as sodium hydroxide or an organic base such as tris (hydroxymethyl) aminomethane or triethanolamine.

However, it is not possible to prepare satisfactory pharmaceutical gels for topical administration by such methods unless the active ingredient is non-reactive with the other components of the gel formulation. For example, problems of storage stability can arise if attempts are made to prepare aqueous gels with hydrolysis-susceptible therapeutic agents. This problem can be illustrated with reference to Varidase (trade mark) which is a mixture of streptokinase and streptodornase useful in the treatment of suppurative surface lesions such as ulcers, pressure sores and infected wounds, of burns and in skin grafting surgical procedures. Varidase is not only moisture sensitive but also can lose its activity at room temperature, and therefore is currently presented as a dry sterile powder in a vial which is stored, away from light, under refrigeration (2°-8°C) for reconstitution by mixing with sterile physiological saline or with a preformed jelly at the time of use. This procedure is manifestly inconvenient, especially in the treatment of patients in their own homes, and moreover leads to problems of ensuring sterility of the topical preparation at the point of administration.

There are many other therapeutically active agents which it would be desirable to administer as gels but which give rise to similar formulation and presentation problems, for example certain antibiotics such as penicillins, certain steroids and certain vitamins.

The present invention seeks to make it easier to administer in gel form therapeutic agents which cannot normally be presented in this form, for example, as in the case of Varidase, because of severe storage instability. However, as will become apparent, the teachings of this invention may also, with advantage, be applied to therapeutic agents with which there are few, if any, problems of presentation in gel form, that is to say it is not limited to use with active ingredients which cannot readily be formulated as gels.

Broadly, the present invention provides a multicompartment pack for the administration topically of a unit dosage form of a therapeutic substance as a gel, said pack containing, in sterile form where required:

(A) either (i) a solution of a pharmaceutically acceptable gel-forming polymer, or (ii) separate polymer and solvent components which when admixed form such a solution;

(B) an agent for gelation of said solution of said gel-forming polymer; and

(C) a unit dosage amount of said therapeutic substance; said components (A), (B) and (C) being contained within at least two separate compartments within the pack such that at least component (B) is maintained out of contact with component (A), and each compartment of the pack is separated from its adjacent compartment or compartments by a frangible barrier, whereby all the components can be admixed together by rupturing the or each barrier just prior to administration to form a gel containing said therapeutic substance.

In a preferred embodiment of the present invention we provide a two-compartment pack for the administration topically of a unit dosage form of a therapeutic substance as a gel, one compartment containing a solution of a pharmaceutically acceptable gel-forming polymer and the other compartment containing the required dose of the therapeutic substance and an agent for gelation of said polymer, and said compartments being separated by a frangible barrier, whereby the components of the two compartments can be admixed together by rupturing the barrier just prior to administration to form a gel containing said therapeutic substance.

The present invention permits therapeutic substances which, to a greater or lesser extent, are reactive with other components of a topical gel to be more readily administered in a gel form, since it becomes possible to·keep apart the therapeutic substance from another component with which it would normally react until the time of use, for example water in the case of a hydrolytically sensitive therapeutic substance to be formulated as an aqueous gel.

Of course, for storage stability, it is necessary to place within separate compartments within the pack any components which, if placed together in a single compartment could inter-react to result in deterioration in properties. For example, in the preferred two compartment pack defined above, it is necessary for storage stability for the therapeutic substance to be inert with respect to the agent for gelation. Alternatively, and in accordance with the teachings of this invention, a third compartment could be provided for the agent for gelation, this third compartment likewise being separated from one or other or both of the other two compartments by a frangible barrier.

Whilst there is theoretically no limit on the number of individual compartments which can be provided within the pack, for reasons of manufacturing cost and ease of manipulation by the user primarily, it is preferred that there should be not more than three separate compartments, and most preferably the components are contained within two compartments only where that is possible.

The preferred pack of this invention is formed from a suitable flexible film material, such as a plastics film material, which is sealed around its peripheral edges to form a closed, flexible envelope, opposed faces of which are sealed together along one or more seal lines extending between opposed sides of the envelope to form the required number of individual compartments for the components of the gel, the components being placed in turn in each compartment before the envelope is finally closed. Methods for the manufacture of such packs are per se known in the art, see for example U.S. Patent No. 3,608,709 issued 28th September 1971. In packs of the present invention, however, it is often important that the gel containing the active ingredient should be sterile for administration, which requires that the internal walls of the resulting envelope or sachet, as well as the components within the compartments thereof, should all be sterile. Sterility sometimes can be achieved by sterilizing the closed sachet at the end of the manufacturing operation using conventional sterilizing techniques such as γ-irradiation, and where possible this is the preferred method of sterilizing. However, in many cases such terminal sterilization cannot be used because of degradation problems. In these instances, therefore, it is necessary to pre-sterilize all the materials and to form the sachet or envelope under aseptic conditions to avoid re-contamination.

The seal lines separating the compartments within the envelope or sachet can be readily ruptured to allow the components to mix together to form a gel at the time of use simply by folding or otherwise manipulating each compartment until the pressure within it is sufficient to cause the seal line or lines separating it from the adjacent compartment or compartments to rupture. As will be appreciated, this manipulation does not jeopardize the sterility of the components within the envelope. When all the barriers separating the compartments have been ruptured in this manner, further manipulation of the envelope ensures that the components are thoroughly and effectively admixed, and once the gel has formed within the envelope, normally after two or three minutes, it can be dispensed by simply cutting open the envelope, eg at one corner.

Preferred plastics film materials for the manufacture of such envelopes are air and moisture-impermeable plastics films which are sealable by conventional techniques such as heat-sealing. Particularly useful are the multi-layer plastics film laminates, for example the commercially available triple layer laminates of "Terphane"/"Saranex"/low density polyethylene ("Terphase" is a co-extrudate of polyethylene terephthalate and polyvinylidine chloride and "Saranex" is a co-extrudate of polyvinylidine chloride and low density polyethylene). Such laminate sheet material can be formed into multi-compartment envelopes preferably using techniques such as taught in U.S. Patent No. 3,608,709 supra. The selection in any particular case of the plastics film material, thickness of film, and so on, will be governed in part by the substances to be contained within the envelope and in part by the requirement that the finished package should be readily manipulatable by the user to form the gel for dispensing.

A number of different pharmaceutically acceptable gel-forming polymers are known in the art, for forming non-aqueous as well as aqueous gels. For most purposes we prefer to use polymers forming aqueous gels, to avoid problems often associated with organic solvents, such as difficulties in handling these solvents, their toxicity, their leaching effects on certain plastics film materials, etc. The "Carbopol" carboxy vinyl polymers referred to above are currently most preferred in most instances, the particular grade (essentially the molecular weight) of choice depending on the desired viscosity of the final gel, which typically may range from 200 - 50,000 cPs (Brookfield viscometer at 20 rpm) for ease of application to a patient whilst ensuring adequate contact with the area of the body being treated. As already mentioned, aqueous solutions of carboxy vinyl polymers are convertible into gels by the addition of a base to raise their pH to about 3.5 or above; the pH at which the gel strength is greatest will depend on the therapeutic substance which is present, being for example greatest at about pH 6.7 with "Varidase". Suitable bases for use as agents for gelation with carboxy vinyl polymers are sodium hydroxide, sodium phosphate, tris (hydroxymethyl) aminomethane, triethanolamine and diisopropanolamine, all of which are generally pharmaceutically acceptable in the amounts normally required. However, carboxy vinyl polymers cannot always be used. For example, we have found that such gel-forming polymers are unsuitable when a tetracycline antibiotic is the therapeutic agent because these antibiotics are liable to degrade when the pH of the polymer is adjusted for gel formation.

A preferred embodiment of this invention is a burstable seam sachet containing as the therapeutic substance, in sterile form, a mixture of streptokinase and streptodornase, such as is available from American Cyanamid Company, Wayne, N.J., United States of America under the trade mark "Varidase". It is surprisingly found that, although hitherto it has been recommended to store "Varidase" powder under refrigerated conditions, to achieve stability, the potency loss of the Varidase in the presently preferred sachet is less than 1% per week at 28°C and less than 0.25% per week at 23°C, thereby obviating the need to store the sachets under refrigerated conditions. It is, however, still desirable that the Varidase not be exposed to light, as hitherto, so that if, as is normally preferred, the sachet is formed from a light transmitting material it should be stored in the dark. Alternatively, the sachet could be formed from a light impermeable material (a plastics laminate including an aluminium film would be suitable), but such a sachet would have the disadvantage that the user would be unable to see that reconstitution and gelling had been completed before dispensing the Varidase to a patient.

A normal unit dosage of Varidase for topical administration ranges from 10,000-200,000 streptokinase units, preferably about 100,000 SKU, and from 2,500 to about 50,000 streptodornase units, preferably about 25,000 SDU. Generally, excipients will be incorporated with this therapeutic substance, to act as a bulking agent, absorption enhancer, osmotic pressure modifier, preservative or protective agent. In the preferred aqueous system of this invention, any pharmaceutically acceptable water-soluble inert compounds can be used as excipients for the Varidase, solid polyethylene glycols being especially preferred since they are highly soluble, pharmaceutically acceptable bulking agents which do not impede gelling of carboxyvinyl polymers. The polyethylene glycol is admixed as a powder with the Varidase powder prior to being filled into its compartment in the sachet.

The most preferred embodiment of this invention, for the administration topically of a sterile unit dosage of a Varidase gel, is a two-compartment sachet formed from the commercially available three-layer plastics film material referred to hereinabove and provided with an interlaminate release coating at locations corresponding to the seal lines between the two compartments. One compartment of the sachet contains 0.25-1.5% w/v in 5 ml of water of a gel-forming carboxyvinyl polymer, such as "Carbopol" 940, preferably about 0.75% w/v of the polymer; and the other compartment contains Varidase sufficient to produce a dose of from 10,000-200,000 SKU, preferably about 100,000 SKU, 99.9-95.0% by weight of polyethylene glycol, preferably polyethylene glycol 3400, as bulking agent for the Varidase to minimise dose variability, and an organic base such as tris (hydroxymethyl) aminomethane or triethanolamine, preferably the former, in an amount such that, when the contents of the two compartments are admixed, the resulting gel has pH in the range of 5-9 preferably about pH 6.7 for maximum viscosity and a viscosity in the range of 200-800 cPs (Ferranti-Shirley cone and plate viscometer at 23°C), preferably about 450 cPs. Amounts of tris (hydroxymethyl) aminomethane therefore suitably range from 0.1-5% by weight, preferably about 3.0-3.5% by weight.

In this case it is important that the interior of the compartments of the present pack, and their contents, should be sterile. Sterility is ensured by pre-sterilizing both the material from which the pack is made and the individual substances to be enclosed therein, and then filling and closing the pack under rigorously aseptic conditions. The Varidase is prepared in sterile form, but for the other components of the sachet conventional sterilizing techniques, particularly gamma irradiation, may be used.

The invention is illustrated by the Examples which follow. Viscosity values were obtained using a Ferranti-Shirley cone and plate viscometer at 25°C.

Example 1

A two-compartment burstable seam sachet formed from the previously described commercially available triple-layer plastics film material was used in this experiment. Into one of the compartments of the sachet were dispensed 5.0 ml of a 0.75% w/v aqueous dispersion of a carboxyvinyl polymer ("Carbopol" 940 - B.F. Goodrich), whilst into the other compartment was dispensed 1 g of polyethylene glycol 3400 powder containing 3.3% w/w trishydroxymethyl aminomethane and "Varidase" (registered Trade Mark) powder with a label content of streptokinase equivalent to 50,000 SKUs. The sachet was then closed by heat-sealing the filling openings.

4

By applying pressure to the compartment containing the "Carbopol" dispersion using finger manipulation, the barrier separating the two compartments of the sachet was broken, and further manipulation of the sachet ensured a thorough mixing of the "Carbopol" dispersion with the remaining components. A clear gel was produced which could then be dispensed from the sachet by cutting off one of its corners. The viscosity of the "Varidase"-containing gel was 475 cP, which is highly suitable for topical applications of gels to patients. The pH of the gel was 7.5.

In order to ensure sterility of the "Varidase" gel at the time of use, all the materials of the sachet can be pre-sterilized by gamma irradiation, except for the "Varidase" which must be made as a sterile product, and the filling and subsequent sealing of the sachet should be conducted under rigorously maintained aseptic conditions. It will be appreciated that sterility will then be maintained while the gel is formed since the mixing of the gel-forming components will be effected within the sachet itself and before it is opened to dispense the gel.

Example 2

A number of burstable seam sachets were prepared as in Example 1 except that the "Varidase" used had a label streptokinase content of 100,000 SKU. The resulting sachets were then stored under different temperatures, and the streptokinase content was measured after intervals of 4 and 12 weeks. The results are shown in Table I below:

## TABLE 1

| Storage Conditions | | % Theory Streptokinase |
| --- | --- | --- |
| Temp ($^{o}$C) | Time (weeks) | Assay |
| 28 | 4 | 106 |
| | 12 | 92 |
| 23 | 4 | 98 |
| | 12 | 97 |
| 3 | 4 | 114 |
| | 12 | 115 |

As shown by Table I, there was no substantial loss of streptokinase activity even after storage at 28°C for twelve weeks. The above test thus demonstrates that the present invention obviates the necessity to store "Varidase" under refrigerated conditions prior to dispensing. Moreover, in the present invention the "Varidase" can be readily dispensed as a gel without the cumbersome mixing of the "Varidase" with pre-formed jelly at the time of treatment which has hitherto been necessary, and without the risk of compromising sterility of the final gel formulation.

Comparative Example

A "Varidase"-containing gel was prepared by mixing "Varidase" with a label streptokinase content of 100,00 SKU, with a pre-formed gel consisting of 0.9% carboxyvinyl polymer (Carbopol 940), 0.85% diisopropanolamine and propylene glycol. The resulting gel was then packed into a number of individual containers which were stored at different temperatures. The stability of the gels was determined after intervals of 4 and/or 12 weeks by assaying the streptokinase content. The results are shown in Table 2.

## TABLE 2

| Storage Conditions | | % Theory Streptokinase |
|---|---|---|
| Temp ($^\circ$C) | Time (weeks) | Assay |
| 28 | 12 | 50 |
| 20 | 4 | 89 |
| | 12 | 74 |
| 8 | 4 | 93 |
| | 12 | 67 |

Table 2 shows that the pre-formed "Varidase" gel was susceptible to an unacceptably high loss of streptokinase activity upon storage. Even under refrigeration (8°C) there was a 33% loss of activity after twelve weeks storage.

Example 3

Example 1 was repeated exactly, except that there was used a 0.5% w/v aqueous dispersion of the Carbopol 940.

When the contents of the two compartments of the sachet were mixed there was formed a clear gel with a viscosity of 157 cP and a pH of 7.5.

Examples 4-9

Example 1 was again repeated in all essential respects, with the contents of the two compartments of the sachet (hereinbelow referred to as compartment I and compartment II respectively) being as identified below. Gel properties on mixing of the contents of the two compartments are also reported.

## Example 4

| Compartment I | Compartment II |
|---|---|
| (a) "Varidase" (50,000 SKU) | |
| (b) 0.1g triethanolamine adsorbed onto precipitated silica (Syloid 244) (55:45 w/w) | [10ml] of 1% w/v aqueous dispersion of Carbopol 940 |
| (c) polyethylene glycol 3400 to 1.0g | |

The gel was turbid and viscous

## Example 5

| Compartment I | Compartment II |
|---|---|
| (a) 200 mg minocycline | |
| (b) 400 mg tris-hydroxymethyl aminomethane | 5 ml of 2% w/v aqueous dispersion of Carbopol 940 |
| (c) 400 mg polyethylene glycol 3400 | |

The gel was judged to be of average viscosity and had a pH of 8.6.

Similar gels were also obtained using 400 mg sucrose or 400 mg mannitol in place of the polyethylene glycol 3400.

Example 6

| Compartment I | Compartment II |
|---|---|
| (a) 330 mg biphenylacetic acid, sodium salt | 10 ml of 2% w/v aqueous dispersion of Carbopol 940 |
| (b) 200 mg tris-hydroxymethyl aminomethane | |

A homogeneous, opaque gel with a viscosity of 873 cP and a pH of 7.3 was produced.

Example 7

| Compartment I | Compartment II |
|---|---|
| (a) piperacillin sodium (equivalent to 100 mg piperacillin) | 9.5 ml of 1% w/v aqueous dispersion of Carbopol 940 |
| (b) 400 mg tris-hydroxymethyl aminomethane | |

A clear, colourless gel of pH 9.1 and viscosity of 461 cP was formed.

Example 8

| Compartment I | Compartment II |
|---|---|
| (a) 100 mg cefotaxime | 9.5 ml of 1% w/v aqueous dispersion of Carbopol 940 |
| (b) 400 mg tris-hydroxymethyl aminomethane | |

A clear, slightly yellow gel with a viscosity of 510 cP and a pH of 9.1 was formed.

Example 9

| Compartment I | Compartment II |
|---|---|
| (a) 500,000 units polymyxin B sulphate | 9.5 ml of 1% w/v aqueous dispersion of Carbopol 940 |
| (b) 400 mg tris-hydroxymethyl aminomethane | |

An opaque white gel was produced which had a pH of 9.2 and a viscosity of 430 cP.

Example 10

Following the general procedures of Example 1, two-compartment burstable seam sachets can be prepared containing, in one compartment, 5 ml of an aqueous dispersion of Carbopol 940 (either 1% w/v or 2% w/v), 0.5% w/v metronidazole and a citrate/phosphate buffer, and in the other compartment either 200 mg or 300 mg of tris-hydroxymethyl aminomethane (TRIS).

Table 3 below shows the pH and viscosity of the gels which were formed upon admixture of the Carbopol dispersion and TRIS. In each case the resulting gel was clear.

## TABLE 3

| Concentration of Carbopol 940 | Amount of TRIS (mg) | pH of gel | Viscosity of gel (cP) |
|---|---|---|---|
| 1% w/v | 200 | 8.7 | 169 |
| 1% w/v | 300 | 9.0 | 158 |
| 2% w/v | 200 | 8.0 | 695 |
| 2% w/v | 300 | 8.7 | 707 |

Example 11

Employing the general procedures outlines in Example 1, two-compartment burstable seam sachets can be prepared containing in one compartment 500 mg triamcinolone and either 200 mg, 400 mg or 600 mg of tris-hydroxymethyl aminomethane (TRIS), and in the other compartment 10 ml of either a 0.5% w/v or a 2.0% w/v aqueous dispersion of Carbopol 940.

Table 4 below gives the pH and viscosity of the opaque, homogeneous gels which were formed when the TRIS/triamcinolone was mixed with the Carbopol dispersion.

## TABLE 4

| Concentration of Carbopol 940 | Amount of TRIS (mg) | pH of gel | Viscosity of gel (cP) |
|---|---|---|---|
| 0.5% w/v | 200 | 9.2 | 449 |
| 0.5% w/v | 400 | 9.6 | 449 |
| 0.5% w/v | 600 | 9.8 | 437 |
| 2.0% w/v | 200 | 6.3 | 959 |

## Example 12

This experiment tested the effect of using different grades of polyethylene glycol in a "Varidase"-containing preparation. Gels were prepared by admixing (a) 10 ml of a 0.75% w/v aqueous dispersion of Carbopol 940 with (b) a mixture of "Varidase" (57,500 SKU), 3.3% w/w of tris-hydroxymethyl aminomethane and polyethylene glycol (PEG) of either grade 200, grade 300 or grade 400, to bring the weight of the mixture to 2 g. The pH and viscosity of the clear gels which resulted are given in Table 5.

### TABLE 5

| Grade of PEG | pH of Gel | Viscosity of Gel |
|---|---|---|
| 200 | 6.5 | 2768 cP |
| 300 | 6.5 | 2967 cP |
| 400 | 6.6 | 3029 cP |

## Example 13

In further experiments following the general procedures described above gels were prepared by mixing (a) acetazolamide and tris-hydroxymethyl aminomethane with (b) 10 ml of either a 1% w/v or a 2% w/v aqueous dispersion of Carbopol 940. The amount of acetazolamide was 320 mg but the amount of the TRIS base varied, as shown by Table 6 below which also reports the pH and viscosity values of the opaque white gels which formed.

### TABLE 6

| Concentration of Carbopol 940 | Amount of TRIS (mg) | pH of gel | Viscosity of gel (cP) |
|---|---|---|---|
| 1% w/v | 400 | 8.5 | 274 |
| 2% w/v | 400 | 8.1 | 904 |
| 2% w/v | 600 | 8.5 | 809 |
| 2% w/v | 200 | 6.3 | 959 |

## Claims

1. A multi-compartment pack for the administration topically of a unit dosage form of a therapeutic substance as a gel, said pack containing,
(A) either (i) a solution of a pharmaceutically acceptable gel-forming polymer, or (ii) separate polymer and solvent components which when admixed form such a solution;
(B) an agent for gelation of said solution of said gel-forming polymer; and
(C) a unit dosage amount of said therapeutic substance;
said components (A), (B) and (C) being contained within at least two separate compartments within the pack such that at least component (B) is maintained out of contact with component (A), and each compartment of the pack is separated from its adjacent compartment or compartments by a frangible barrier, whereby all the components can be admixed together by rupturing the or each barrier just prior to administration to form a gel containing said therapeutic substance.

2. A pack according to Claim 1, wherein said therapeutic substance (C) is unstable in the presence of said component (A) and/or component (B) such that its therapeutic activity deteriorates upon storage of mixtures thereof, and wherein component (C) is contained within the pack out of contact with the or each other component with which it is unstable.

3. A pack according to Claim 1 or Claim 2, wherein the interior walls of each compartment of the pack, and the components within each compartment, are sterile.

4. A pack according to any preceding claim wherein the pack is formed from a flexible film material which is sealed around its peripheral edges to form a closed, flexible envelope, opposed faces of which are sealed together along one or more seal lines extending between opposed sides of the envelope to form said compartments, said seal line or lines forming said frangible barrier or barriers.

5. A two-compartment pack according to any preceding claim.

6. A pack according to Claim 5, one compartment containing a solution of a pharmaceutically acceptable gel-forming polymer, and the other compartment containing said required dose of said therapeutic substance and said agent for gelation of the polymer.

7. A pack according to any preceding claim, wherein said solution of said pharmaceutically acceptable gel-forming polymer is an aqueous solution of a high molecular weight carboxyvinyl polymer, and said agent for gelation of said polymer is a base.

8. A pack according to any preceding claim, wherein said therapeutic substance comprises a mixture of streptokinase and streptodornase.

9. A pack for the administration of a unit dosage form of a mixture of streptokinase and streptodornase as a gel, the pack being an internally sterile, flexible envelope internally divided by a pressure-burstable seam into two compartments, the first of which compartments contains a sterile aqueous solution of a carboxyvinyl polymer having a molecular weight of at least one million, and the second of which compartments contains, in sterile form, from 10,000-200,000 units of streptokinase, from 2,500 to about 50,000 units of streptodornase, and an inorganic or organic base in amount sufficient to gel said aqueous polymer solution when said seam separating the two compartments is ruptured by external manipulation of said envelope to permit the contents of the two compartments to become admixed.

10. A pack according to Claim 9, wherein said second compartment further contains a sterile excipient for said streptokinase and streptodornase.